Europäisches Patentamt

European Patent Office

Office européen des brevets

(11) Publication number: **0 256 695 A2**

## (12) EUROPEAN PATENT APPLICATION

(21) Application number: **87306610.4**

(22) Date of filing: **27.07.87**

(51) Int. Cl.⁴: **A61K 35/32 , A61K 6/00**

(30) Priority: **08.08.86 US 894699**

(43) Date of publication of application:
**24.02.88 Bulletin 88/08**

(84) Designated Contracting States:
**DE FR GB IT**

(71) Applicant: **Fugazzotto, Paul A.**
**52 Cushing Road**
**Milton Massachusetts 02186(US)**

(72) Inventor: **Fugazzotto, Paul A.**
**52 Cushing Road**
**Milton Massachusetts 02186(US)**

(74) Representative: **Baverstock, Michael George**
**Douglas et al**
**BOULT, WADE & TENNANT 27 Furnival Street**
**London, EC4A 1PQ(GB)**

(54) Product for use as an osteoinductive agent, and method of preparing same.

(57) A method of preparing dentin for use as an osteoinductive agent includes removing substantially all material other than dentin from a tooth, and grinding the dentin into particles less than one millimeter in size. The particles are treated, such as by freeze-drying, to eliminate antigenic characteristics of the dentin.

FIG. 1

## PRODUCT FOR USE AS AN OSTEOINDUCTIVE AGENT, AND METHOD OF PREPARING SAME

This invention relates to a product for use as an osteoinductive agent, and a method of preparing same. In particular the invention relates to the manufacture of allogenic dentin particles of small size for use in bone regeneration.

A number of experiments have been directed toward osteoinduction, that is, bone regeneration as stimulated by a selected substance. Osteoinduction involves stimulating pluripotential mesenchymal cells into functional osteoblasts. This process appears to be controlled by acid-insoluble proteins known as bone morphogenic proteins.

Freeze-dried bone contains bone morphogenic proteins and for at least two decades has been used in particle form, after reconstitution, as a graft material to stimulate bone regeneration. Allogenic bone is freeze-dried to eliminate the antigenic characteristics of the bone; still, however, the graft material must be obtained from the same species as the recipient. Further, bone particles for osteoinduction in humans must be derived from freshly extracted human bone, typically from organ donors when available. These bone particles are relatively expensive due to the small supply of fresh human bone.

A tooth is composed of enamel, cementum, dentin and pulp tissue. Dentin is suspected also to contain bone morphogenic proteins.

Many osteoinduction studies have utilized pieces of teeth but without significant results. Of the few studies which utilized pure dentin, most were unsuccessful. The sole successful studies involved placement of decalcified or demineralized dentin into muscle tissue, into oral cheek tissue, and into the marrow of long bone. These studies are deemed successful simply because they induced some formation of bone; none were concerned with the interfacing of the formation with existing structure in the body. None of the above-mentioned studies prepared the dentin by freeze-drying it, and none controlled the size of the dentin particles.

A number of people suffer degeneration of the osseous supporting structures of the teeth. Regeneration of the osseous structures is difficult due to the harsh environment of the mouth, e.g., acid by-products of bacteria. Unlike virtually any other region of the body, where for the skeleton and other organs the epithelium serves as a significant barrier to bacteria and other harmful agents, the epithelial barrier is breached in the oral area by the normal protrusion of teeth through the epithelium. Bone surrounding the teeth is also exposed to the rigorous oral cavity environment.

Regeneration of periodontal bone defects can be accomplished using freeze-dried bone as described above. As also noted above, this graft material is expensive.

The other presently available treatment for periodontal bone defects utilizes synthetic grafting material available in resorbable and nonresorbable form. Synthetic material does not contain bone morphogenic protein, however, and is ineffective as a graft material: it simply serves as a filler. Its use continues since it resembles bone radiographically and mechanically arrests penetration of a periodontist's probe. Unfortunately, further degradation of the bone defect often results.

It is an object of this invention to provide for an improved method of osteoinduction in bone defects using freeze-dried dentin.

It is a further object of this invention to provide for such a method which is effective without decalcification or other demineralization of the dentin.

It is a further object of this invention to provide for such a method which can regenerate osseous supporting structures of the teeth.

Yet another object of this invention is to provide for such a method which provides a desirable bone-tooth interface.

It is a further object of this invention to provide for such a method which withstands the rigors of the oral environment.

A still further object of this invention is to provide an improved method of preparing dentin for use in osteoinduction.

Yet another object of this invention is to provide unique dentin particles.

This invention results from the realization that a truly effective substance for use as an osteoinductive agent can be achieved by obtaining dentin from a tooth, grinding the dentin into particles preferably less than one millimeter in size, and treating the dentin particles to eliminate antigenic characteristics, such as by freeze-drying the dentin.

This invention features a method of preparing dentin for use as an osteoinductive agent. The method includes separating dentin from the remainder of a tooth, grinding the dentin into particles less than one millimeter in size, and treating the dentin particles to eliminate antigenic characteristics of the dentin.

In one embodiment, the method further includes sterilizing the dentin particles without damaging proteins within the dentin, such as by exposing the dentin particles to ethylene gas. The dentin may be ground to particles preferably 300 to 800 micrometers in size, and treated by freeze-drying;

the tooth from which the dentin is obtained may be a freshly extracted human tooth. Also claimed are the treated and freeze-dried dentin particles prepared by these methods.

This invention also enables a method of osteoinduction including exposing the surface of a defect in a bone to be regenerated and applying treated dentin particles, of less than one millimeter in size, to the bone defect.

The exposing step may include cleaning the surface to remove soft tissue from it. The dentin particles may be freeze-dried and reconstituted in a water-based liquid such as a sterile saline solution or distilled water. The particles range in size from 300 to 800 micrometers.

The dentin may be at least partially enclosed with soft tissue surrounding the bone defect and microfibrillar collagen may be placed over the dentin. The bone may be human periodontal bone and the dentin is obtained from human teeth.

Other objects, features and advantages of the invention will occur from the following description and the accompanying drawing, which is a cross-sectional view of a vital incisor tooth supported by peridontal bone containing a bone defect.

Dentin particles according to this invention are prepared by extracting a vital tooth and removing all enamel, cementum and pulp tissue using a high-speed rotary instrument. The pure dentin is then conventionally ground into particles and filtered: particles less than one millimeter in size are acceptable; particles ranging in size from 300-800 μm are desirable; and particle sizes of 400-600 μm are preferred.

The dentin particles are then conventionally freeze-dried such as described for bone particles in Hurt, W., "Freeze-dried bone homografts in periodontal lesions in dogs," J. Periodontol. 39:89 (1968). Alternatively, the intact dentin can be freeze-dried before fragmentation. Freeze-drying eliminates the antigenic characteristics of the dentin particles to minimise possible rejection by the recipient. Other treatment which eliminates antigenic characteristics while not harming proteins within the dentin is also acceptable.

Dentin particles prepared in this manner are effective without decalcification or demineralization. However, the calcium or other minerals can be removed by conventional techniques from the dentin, and the dentin otherwise prepared and utilized according to this invention.

To prevent possible contamination from use of the dentin particles, the freeze-dried particles are conventionaly gas sterilized in ethylene gas such as described in Hurt, supra. Any sterilization procedure is acceptable which does not damage proteins within the dentin, particularly the bone morphogenic proteins; present heat or radiation sterilization is not acceptable for that reason.

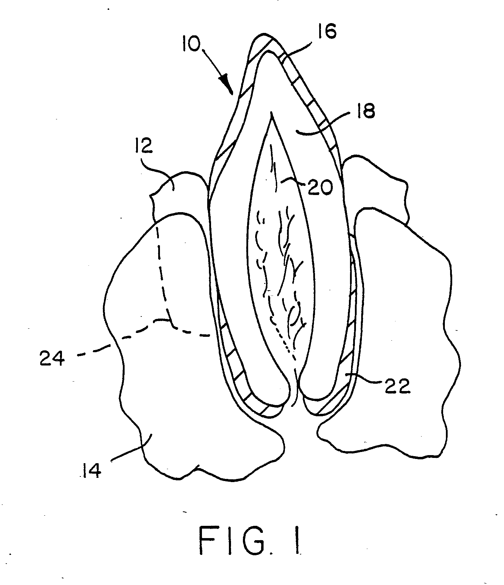

These dentin particles are utilized to induce bone regeneration where desired. It is particularly suitable for regenerating defects in periodontal bones proximate teeth such as tooth 10, Fig. 1, shown in relation to gingiva 12 and periodontal bone 14. Tooth 10 is composed of enamel 16, dentin 18, pulp cavity 20 and cementum 22. Defect 24, shown in phantom in bone 14, represents a bone defect.

The procedure of regenerating the bone defect includes exposing the surface of the defect and removing soft tissue from its surface. The cleaning of the surface to remove the bacteria, loose tissue, and other matter can be accomplished by mechanical scraping or by applying ultra sound to the surface. In the illustrative examples described below, all bone defects were degranulated and decorticated with high-speed rotary burrs and root surfaces were planed, both manually and with high-speed rotary instruments.

Prior to implantation of the dentin particles, the freeze-dried particles are reconstituted by wetting them in sterile saline solution or distilled water, e.g. for 20 minutes. Other water-based liquids such as saliva or serum could be used, particularly the saliva of the recipient. Actual hydration of the dentin particles may occur, or the particles may simply be wetted without chemically combining with the water; in either case the wetted particles are easier to manipulate and remain more readily in position once emplaced. After wetting by the liquid, the dentin particles are gently placed with a curette to the brims of the bone defects. Minimum compressive force is applied to the dentin particles to encourage the penetration of blood vessels.

In the examples below the bone defects were revealed by surgically creating a split or full-split apically positioned mucoperiosteal flap buccally and a split-thickness crestally anticipated flap lingually. The flaps were repositioned over the dentin particles employing periosteal suturing with 4.0 silk. It is desirable to place microfibrillar collagen such as Avitene, available from Avicon, Inc. Humacao, Puerto Rico, over the dentin particles and against adjacent bone to encourage clotting and coverage of the graft material.

The following examples are illustrative of the use of the product of the invention in osteoinduction.

## EXAMPLE 1

Upon probing the buccal surface of the mandibular left lateral incisor of a male in his mid-thirties, the probe descended 10 millimeters. The incisor exhibited a class III mobility and very little attached keratinized tissue. There was profuse bleeding upon probing. When the mucoperiosteal flap was reflected, a severe buccal dehiscence was revealed. The dehiscence involved exposure of a major portion of the root and terminated in a 2 mm moat-like defect extending onto the mesial and distal surfaces of the tooth. Bone degeneration was so extensive that conventional teachings would strongly suggest extracting the tooth without attempting to preserve it. The dehiscence and defect were filled with freeze-dried dentin prepared according to this invention, and the soft tissue flap was placed at the level of the adjacent osseous crests by suturing.

At seven months postoperatively, a probing depth of 2 to 3 mm was present. A re-entry procedure revealed osseous regeneration of a major portion of the buccal plate of the mandibular left lateral incisor. The mobility of the tooth was now less than Class I. One year postoperatively, the area was healthy and stable.

## EXAMPLE 2

A female in her late fifties exhibited a 9 mm probing on the mesial surface of the mandibular left canine. Following reflection of a partial thickness mucoperiosteal flap, a widemouthed, three-wall osseous defect of between 6 and 8 mm in depth was revealed. The bone defect was filled to its mouth with freeze-dried dentin according to this invention and covered with Avitene. The buccal flap was apically positioned at the osseous crest. The mucoperiosteal flap did not cover the graft material interproximally, but coverage was provided by the Avitene.

Visual examination five weeks postoperatively confirmed that the area was healing nicely. After a six-month period, surgical re-entry revealed a narrow, three-wall defect less than 2 mm in depth.

These examples are two of at least sixteen cases in which six or more months have elapsed postoperatively. Positive results have been achieved in all cases to date.

## Claims

1. A method of preparing dentin for use as an osteoinductive agent, which method comprises separating dentin from the remainder of a tooth; grinding the dentin into particles less than one millimeter in size; and treating the dentin particles to eliminate antigenic characteristics of the dentin.

2. A method as claimed in claim 1 further including sterilizing the dentin particles without damaging proteins within the dentin.

3. A method as claimed in claim 2 in which the sterilizing includes exposing the dentin particles to ethylene gas.

4. a method as claimed in any one of the preceding claims in which the treating includes freeze-drying the dentin particles.

5. A method as claimed in any one of the preceding claims in which the dentin is ground to particles 300 to 800 micrometers in size.

6. A method as claimed in any one of the preceding claims in which the tooth is a freshly extracted human tooth.

7. Treated dentin particles wherein prepared by a method as claimed in any one of the preceding claims.

8. A product for use as an osteoinductive agent whenever comprising treated dentin particles as claimed in claim 7.

9. A product for use as an osteoinductive agent whenever comprising freeze-dried dentin particles.

10. A product as claimed in claim 9 in which the particles are less than one millimeter in size.

FIG. 1